Europäisches Patentamt

European Patent Office

Office européen des brevets

(11) Veröffentlichungsnummer: **0 258 543**
A2

# (12) EUROPÄISCHE PATENTANMELDUNG

(21) Anmeldenummer: 87107998.4

(22) Anmeldetag: 03.06.87

(51) Int. Cl.4: **G01N 27/56** , F02D 41/00

(30) Priorität: 23.08.86 DE 3628707
03.11.86 DE 3637304

(43) Veröffentlichungstag der Anmeldung:
09.03.88 Patentblatt 88/10

(84) Benannte Vertragsstaaten:
DE FR GB SE

(71) Anmelder: VDO Adolf Schindling AG
Gräfstrasse 103
D-6000 Frankfurt/Main(DE)

(72) Erfinder: Blümel, Thomas
Zum Feldberg 49
D-6384 Schmitten 1(DE)
Erfinder: Collonia, Harald
1773 Star-Batt Drive P.O. Box 2897
Rochester, MI 48063(DE)

(74) Vertreter: Klein, Thomas, Dipl.-Ing. (FH)
Sodener Strasse 9 Postfach 6140
D-6231 Schwalbach a. Ts.(DE)

(54) Verfahren und Schaltungsanordnung zur Erkennung der Betriebsbereitschaft einer Sauerstoffmesssonde.

(57) Bei einem Verfahren zur Erkennung der Betriebsbereitschaft einer Sauerstoffmeßsonde, die im Abgaskanal einer Brennkraftmaschine angeordnet ist und zusammen mit einer Regeleinrichtung zur Regelung der Gemischaufbereitung für die Brennkraftmaschine dient, wird die an der Sauerstoffmeßsonde anliegende Spannung nacheinander für zwei verschiedene Belastungszustände gemessen und aus den Meßergebnissen der Innenwiderstand errechnet, der mit einem vorgegebenen Wert verglichen wird.

FIG. 1

## Verfahren und Anordnung zur Erkennung der Betriebsbereitschaft einer Sauerstoffmeßsonde

Die Erfindung betrifft ein Verfahren und eine Anordnung zur Erkennung der Betriebsbereitschaft einer Sauerstoffmeßsonde, die im Abgaskanal einer Brennkraftmaschine angeordnet ist und zusammen mit einer Regeleinrichtung zur Regelung der Gemischaufbereitung für die Brennkraftmaschine dient. Zur Erzielung möglichst schadstofffreier Abgase sind Regeleinrichtungen für Brennkraftmaschinen bekannt, bei denen der Sauerstoffgehalt im Abgaskanal gemessen und ausgewertet wird. Hierzu sind Sauerstoffmeßsonden, sogenannte Lambda-Sonden, bekannt, die nach dem Prinzip der Ionenleitung durch einen Festelektrolyten in Folge einer Sauerstoffpartialdruckdifferenz arbeiten und entsprechend dem im Abgas vorliegenden Sauerstoffpartialdruck ein Spannungssignal abgeben, das beim Übergang vom Sauerstoffmangel zum Sauerstoffüberschuß einen Spannungssprung aufweist.

Der Innenwiderstand der bekannten Sauerstoffmeßsonden ist jedoch bei geringen Temperaturen derart groß, daß das von der Sauerstoffmeßsonde abgegebene Signal nach einem Kaltstart und während einer Warmlaufphase der Brennkraftmaschine nicht ausgewertet werden kann. Bei bekannten Einrichtungen zur Regelung der Gemischaufbereitung ist daher bis zum Bereich der Betriebsbereitschaft der Sauerstoffmeßsonde eine vom Ausgangssignal der Sauerstoffmeßsonde unabhängige Steuerung vorgesehen. Erst wenn die Sauerstoffmeßsonde ihre Betriebsbereitschaft erreicht hat, wird deren Ausgangssignal zur Regelung des Brennstoff-Luftverhältnisses herangezogen.

Bei einem bekannten Verfahren zur Überwachung der Betriebsbereitschaft einer Sauerstoffmeßsonde wird die Sauerstoffmeßsonde mit einer Prüfspannung mit einem konstanten mittleren Wert der von der Sauerstoffmeßsonde erzeugbaren Spannung belastet. Die resultierende Spannung am Sondenausgang wird als Kontrollgröße der Betriebsbereitschaft zwei Vergleichsvorrichtungen zum Vergleich mit einem oberen und einem unteren Sondenwert jeweils entsprechend einer Mindestausgangsspannung der Sauerstoffmeßsonde zugeführt, und über ein Zeitglied wird eine Gemischsteuervorrichtung anstelle der Gemischregeleinrichtung entsprechend dem Ausgangssignal der Vergleichsvorrichtung zu-oder abgeschaltet.

Bei einem anderen bekannten Verfahren zur Überwachung der Betriebsbereitschaft einer Sauerstoffmeßsonde, bei welcher ebenfalls eine unter dem Einfluß des Sondenverhaltens resultierende Spannung von zwei Vergleichsschaltungen mit Schwellwertspannungen abgetastet und anschließend ausgewertet wird, wird zur Erfassung des die Sondenbereitschaft kennzeichnenden Sonden-Innenwiderstandes der Sauerstoffmeßsonde eine konstante Bezugsspannung entgegengeschaltet, wobei die der sich hierdurch ergebenden Ausgangsspannung entgegengeschalteten Schwellwertspannungen der Vergleichsschaltungen um vorgegebene Differenzwerte oberhalb und unterhalb der Bezugsspannung liegen, dabei werden die von den Vergleichsschaltungen abgegebenen, insgesamt drei logischen Schaltzuständen entsprechende Ausgangssignale von einer nachgeschalteten Auswerteschaltung zur Umschaltung zwischen Regelung auf Steuerung bzw. umgekehrt verarbeitet.

Aufgabe der vorliegenden Erfindung ist es, ein Verfahren zur Erkennung der Betriebsbereitschaft einer Sauerstoffmeßsonde anzugeben, welches in zuverlässiger Weise mit einfachen und preiswerten Anordnungen durchgeführt werden kann. Außerdem soll durch die Erfindung ermöglicht werden, die Erkennung mit einfachen Mitteln an die jeweils vorliegenden Verhältnisse anzupassen.

Das erfindungsgemäße Verfahren ist dadurch gekennzeichnet, daß die an der Sauerstoffmeßsonde anliegende Spannung nacheinander für zwei verschiedene Belastungszustände gemessen wird und daß aus den Meßergebnissen der Innenwiderstand errechnet und mit einem vorgegebenen Wert verglichen wird.

Das erfindungsgemäße Verfahren kann nicht nur zur Überwachung der Sauerstoffmeßsonde während der Warmlaufphase, sondern auch zur Überwachung während des Betriebes angewendet werden.

Durch das erfindungsgemäße Verfahren erfolgt eine genaue Messung des Innenwiderstandes. Durch den anschließenden Vergleich mit dem vorgegebenen Wert ist ein klares Kriterium für die Betriebsbereitschaft gegeben. Der vorgegebene Wert kann insbesondere dann leicht den Verhältnissen im Einzelfall angepaßt werden, wenn er gemäß einer Weiterbildung der Erfindung in einem Speicher abgelegt ist, dessen Inhalt veränderbar ist.

Gemäß einer anderen Weiterbildung der Erfindung ist vorgesehen, daß die verschiedenen Belastungszustände dadurch erzielt werden, daß eine Reihenschaltung aus der Sauerstoffmeßsonde und einem Widerstand abwechselnd mit zwei verschiedenen Spannungen beaufschlagt wird. Dabei besteht eine vorteilhafte Ausführungsform darin, daß die eine Spannung 0 V beträgt und daß die andere Spannung eine Referenzspannung ist. Vorzugsweise ist vorgesehen, daß die Referenzspannung ferner einem Analog/Digital-Wandler zugeführt wird.

Eine vorteilhafte Anordnung zur Durchführung des erfindungsgemäßen Verfahrens besteht darin, daß ein erster Anschluß der Sauerstoffmeßsonde mit konstantem Potential und ein zweiter Anschluß über einen Widerstand mit einer von einem Mikrocomputer schaltbaren Spannungsquelle verbunden ist und daß an den zweiten Anschluß ferner der Eingang eines Analog/Digital-Wandlers angeschlossen ist, der dem Mikrocomputer zugeordnet ist Bei entsprechender Programmierung kann ein ohnehin für Regelzwecke eingesetzter Mikrocomputer zur Durchführung des erfindungsgemäßen Verfahrens durch Hinzufügung von nur wenigen elektronischen Elementen eingesetzt werden.

Die derzeit auf dem Markt erhältlichen Mikrocomputer mit integriertem Analog/Digital-Wandler weisen Eingangswiderstände auf, welche nicht größer als die der Sauerstoffmeßsonde im kalten Zustand sind. Um die Verwendung dieser auf dem Markt erhältlichen Mikrocomputer zu ermöglichen, ist gemäß einer Weiterbildung vorgesehen, daß zwischen dem zweiten Anschluß der Sauerstoffmeßsonde und dem Eingang des Analog/Digital-Wandlers ein Impedanz-Wandler angeordnet ist.

Eine besonders einfache Ausführungsform der Anordnung zur Durchführung des erfindungsgemäßen Verfahrens besteht darin, daß die - schaltbare Spannungsquelle im wesentlichen aus einem Transistor, dessen Emitter mit dem konstanten Potential beaufschlagt ist, und einem Kollektorwiderstand besteht, dessen vom Kollektor abgewandter Anschluß an eine Spannungsquelle angeschlossen ist.

Das erfindungsgemäße Verfahren ist weiterhin dadurch gekennzeichnet, daß die verschiedenen Belastungszustände dadurch erzielt werden, daß die Sauerstoffmeßsonde abwechselnd mit einem ersten Widerstand und mit der Parallelschaltung aus dem ersten und einem zweiten Widerstand belastet wird. Dadurch sind einfache Schaltungsanordnungen zur Durchführung des Verfahrens möglich.

Der vorgegebene Wert kann insbesondere dann leicht den Verhältnissen im Einzelfall angepaßt werden, wenn er gemäß einer Weiterbildung des erfindungsgemäßen Verfahrens einem Speicher entnommen wird, dessen Inhalt veränderbar ist.

Eine vorteilhafte Schaltungsanordnung zur Durchführung des erfindungsgemäßen Verfahrens ist dadurch gekennzeichnet, daß der Sauerstoffmeßsonde ein erster Widerstand und eine Reihenschaltung aus einem zweiten Widerstand und einem von einem Mikrocomputer steuerbaren Schalter parallelgeschaltet ist und daß an die Sauerstoffmeßsonde ferner der Eingang eines Analog/Digital-Wandlers angeschlossen ist, der dem Mikrocomputer zugeordnet ist.

Bei entsprechender Programmierung kann ein ohnehin für Regelzwecke eingesetzter Mikrocomputer zur Durchführung des erfindungsgemäßen Verfahrens durch Hinzufugung von nur wenigen elektronischen Bauelementen verwendet werden.

Die derzeit auf dem Markt erhältlichen Mikrocomputer mit integriertem Analog/Digital-Wandler weisen Eingangswiderstände auf, welche nicht größer als die der Sauerstoffmeßsonde im kalten Zustand sind. Um die Verwendung dieser auf dem Markt erhältlichen Mikrocomputer zu ermöglichen, ist gemäß einer Weiterbildung vorgesehen, daß zwischen dem zweiten Anschluß der Sauerstoffmeßsonde und dem ·Eingang des Analog/Digital-Wandlers ein Impedanz-Wandler angeordnet ist.

Eine besonders einfache Ausführungsform der Schaltungsanordnung zur Durchführung des erfindungsgemäßen Verfahrens besteht darin, daß der steuerbare Schalter im wesentlichen aus einem Transistor besteht.

Die Erfindung läßt zahlreiche Ausführungsformen zu. Zwei davon sind schematisch in der Zeichnung dargestellt und nachfolgend beschrieben.

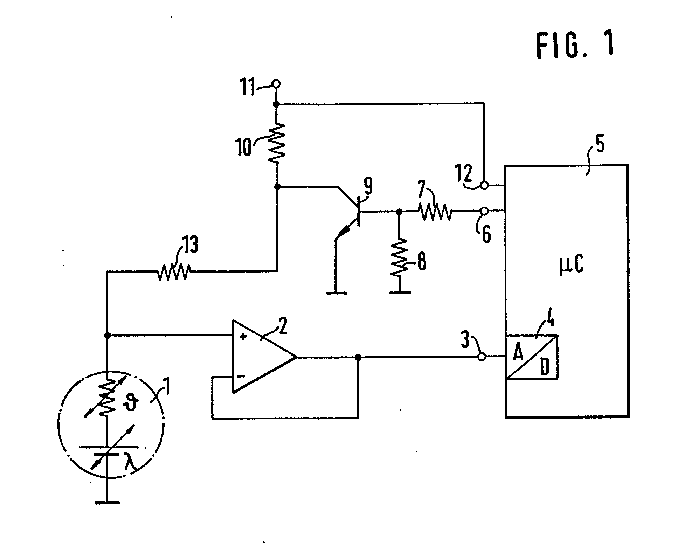

Die an sich bekannte Sauerstoffmeßsonde ist in der Figur1 lediglich als eine Spannungsquelle, deren Spannung von dem Sauerstoffanteil abhängt und deren Innenwiderstand $R_i$ temperaturabhängig ist, dargestellt. Die Ausgangsspannung der Sauerstoffmeßsonde wird über einen als Impedanz-Wandler geschalteten Operationsverstärker 2 einem Eingang 3 eines Analog-Digital-Wandlers 4, der Teil eines Mikrocomputers 5 ist, zugeführt. An einem Ausgang 6 des Mikrocomputers ist über einen Spannungsteiler 7, 8 die Basis eines Transistors 9 angeschlossen. Der Emitter des Transistors 9 ist mit Massepotential verbunden, während der Kollektor des Transistors 9 über einen Kollektor-Widerstand 10 mit einem Anschluß 11 einer Spannungsquelle verbunden ist, welche auch als Referenzspannung für den Analog/Digital-Wandler 4 dient und deshalb mit einem weiteren Eingang 12 des Mikrocomputers 5 verbunden ist. Ferner ist die Sauerstoffmeßsonde 1 über einen Widerstand 13 an den Kollektor des Transistors 9 angeschlossen.

Durch ein im Mikrocomputer 5 gespeichertes Programm wird nun zur Ermittlung der Betriebsbereitschaft der Sauerstoffmeßsonde der Transistor 9 abwechselnd in den nichtleitenden und in den leitenden Zustand gesteuert. Ist der Transistor 9 nichtleitend, so wird die Reihenschaltung aus der Sauerstoffmeßsonde 1 und dem Widerstand 13 mit einer Spannung beaufschlagt, die etwa der bei 11 zugeführten Referenzspannung entspricht, da der Wert des Widerstandes 10 wesentlich kleiner als der Wert des Widerstandes 13 ist.

Der Widerstand 13 ist derart dimensioniert, daß er einerseits eine zuverlässige Messung des Innenwiderstandes der Sauerstoffmeßsonde 1 ermöglicht und andererseits bei der Auswertung des Sondensignals im betriebsbereiten Zustand keine wesentliche Verfälschung des Sondensignals verursacht. Bei üblicherweise verwendeten Sauerstoffmeßsonden beträgt der Innenwiderstand im kalten Zustand etwa 15 MOhm, während er im Betriebszustand etwa 10 kOhm oder kleiner ist. Für den Wert des Widerstandes 13 hat sich daher ein Wert von etwa 1 MOhm als günstig herausgestellt, bei dem einerseits eine einwandfreie Messung des Innenwiderstandes vor der Betriebsbereitschaft und ande rerseits keine merkbare Verfälschung des Ausgangssignals der Sauerstoffmeßsonde vorliegt. Außerdem wird durch einen Widerstand 13 mit diesem Wert die Sauerstoffmeßsonde nicht über ihre zulässigen Toleranzen hinaus belastet.

Ist der Transistor leitend, so wird die Sauerstoffmeßsonde mit dem Widerstand 13 belastet. Durch Messung der Spannung an der Sauerstoffmeßsonde 1 in beiden Fällen kann mit Hilfe des Mikrocomputers in einfacher Weise der Innenwiderstand nach der Gleichung

$$R_i = R_L \frac{V_H - V_L}{V_{ref} - V_H + V_L}$$

errechnet werden. Dieser Wert wird anschließend mit einem gespeicherten Wert verglichen. Ist der ermittelte Wert kleiner als der gespeicherte Wert, so wird die von der Sauerstoffmeßsonde 1 abgegebene und über den Impdanz-Wandler 2 dem Analog/Digital-Wandler 4 zugefuhrte Spannung zur Regelung der Gemischaufbereitung mit Hilfe des Mikrocomputers 5 herangezogen.

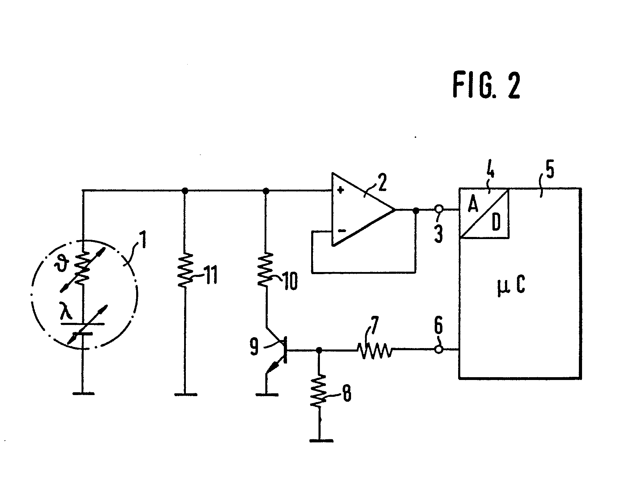

Die an sich bekannte Sauerstoffmeßsonde 1 ist in der Figur2 lediglich als eine Spannungsquelle, deren Spannung von dem Sauerstoffanteil abhängt und deren Innenwiderstand $R_i$ temperaturabhängig ist, dargestellt. Die Ausgangsspannung der Sauerstoffmeßsonde 1 wird über einen als Impedanz-Wandler geschalteten Operationsverstärker 2 einem Eingang 3 eines Analog-Digital-Wandlers 4, der Teil eines Mikrocomputers 5 ist, zugeführt. An einem Ausgang 6 des Mikrocomputers ist über einen Spannungsteiler 7, 8 die Basis eines Transistors 9 angeschlossen. Der Emitter des Transistors 9 ist mit Massepotential verbunden, während der Kollektor des Transistors 9 uber einen Widerstand 10 mit der Sauerstoffmeßsonde verbunden ist. Ferner ist an die Sauerstoffmeßsonde 1 ein weiterer Widerstand 11 angeschlossen.

Durch ein im Mikrocomputer 5 gespeichertes Programm wird nun zur Ermittlung der Betriebsbereitschaft der Sauerstoffmeßsonde der Transistor 9 abwechselnd in den nichtleitenden und in den leitenden Zustand gesteuert. Ist der Transistor 9 nichtleitend, so wird die Sauerstoffmeßsonde 1 lediglich mit dem Widerstand 11 belastet. Ist der Transistor leitend, ist die Belastung durch die Parallelschaltung beider Widerstände gegeben. Aus dem Verhältnis der Ausgangsspannungen der Sauerstoffmeßsonde bei beiden Belastungen wird der Innenwiderstand $R_i$ berechnet. Dabei ist die Messung genügend schnell, damit sich während eines Meßzyklus die Ausgangsspannung praktisch nicht ändert.

Die Widerstände 10 und 11 sind derart dimensioniert, daß einerseits eine zuverlässige Messung des Innenwiderstandes der Sauerstoffmeßsonde 1 ermöglicht und andererseits bei der Auswertung des Sondensignals im betriebsbereiten Zustand keine wesentliche Verfälschung des Sondensignals verursacht wird. Bei üblicherweise verwendeten Sauerstoffmeßsonden beträgt der Innenwiderstand im kalten Zustand etwa 15 MOhm, während er im Betriebszustand etwa 10 kOhm oder kleiner ist. Für die Widerstände 10 und 11 hat sich daher ein Wert von etwa 1 MOhm als günstig herausgestellt, bei dem einerseits eine einwandfreie Messung des Innenwiderstandes vor der Betriebsbereitschaft und andererseits keine merkbare Verfälschung des Ausgangssignals der Sauerstoffmeßsonde vorliegt.

Der für den Innenwiderstand ermittelte Wert wird anschließend mit einem gespeicherten Wert verglichen. Ist der ermittelte Wert kleiner als der gespeicherte Wert, so wird die von der Sauerstoffmeßsonde 1 abgegebene und über den Impedanz-Wandler 2 dem Analog-Digital-Wandler 4 zugefuhrte Spannung zur Regelung der Gemischaufbereitung mit Hilfe des Mikrocomputers 5 herangezogen.

## Ansprüche

1. Verfahren zur Erkennung der Betriebsbereitschaft einer Sauerstoffmeßsonde, die im Abgaskanal einer Brennkraftmaschine angeordnet ist und zusammen mit einer Regeleinrichtung zur Regelung der Gemischaufbereitung fur die Brennkraftmaschine dient, dadurch gekennzeichnet, daß die an der Sauerstoffmeßsonde anliegende Spannung nacheinander für zwei verschiedene Belastungszustände gemessen wird und daß aus den Meßergebnissen der Innenwiderstand errechnet und mit einem vorgegebenen Wert verglichen wird.

2. Verfahren nach Anspruch 1, dadurch gekennzeichnet, daß die verschiedenen Belastungszustände dadurch erzielt werden, daß eine Reihenschaltung aus der Sauerstoffmeßsonde und einem Widerstand abwechselnd mit zwei verschiedenen Spannungen beaufschlagt wird.

3. Verfahren nach Anspruch 2, dadurch gekennzeichnet, daß die eine Spannung 0 V beträgt und daß die andere Spannung eine Referenzspannung ist.

4 Verfahren nach Anspruch 3, dadurch gekennzeichnet, daß die Referenzspannung ferner einem Analog/Digital-Wandler zugeführt wird.

5. Verfahren nach einem der vorhergehenden Ansprüche, dadurch gekennzeichnet, daß der vorgegebene Wert einem Speicher entnommen wird, dessen Inhalt veränderbar ist.

6. Anordnung zur Durchführung des Verfahrens nach einem der vorhergehenden Ansprüche, dadurch gekennzeichnet, daß ein erster Anschluß der Sauerstoffmeßsonde (1) mit konstantem Potential und ein zweiter Anschluß über einen Widerstand (13) mit einer von einem Mikrocomputer (5) - schaltbaren Spannungsquelle (6 bis 11) verbunden ist und daß an den zweiten Anschluß ferner der Eingang eines Analog/Digital-Wandlers (4) angeschlossen ist, der dem Mikrocomputer (5) zugeordnet ist.

7. Anordnung nach Anspruch 6, dadurch gekennzeichnet, daß zwischen dem zweiten Anschluß der Sauerstoffmeßsonde (1) und dem Eingang des Analog/Digital-Wandlers (4) ein Impedanz-Wandler (3) angeordnet ist.

8. Anordnung nach einem der Ansprüche 6 oder 7, dadurch gekennzeichnet, daß die - schaltbare Spannungsquelle (6 bis 11) im wesentlichen aus einem Transistor (9), dessen Emitter mit dem konstanten Potential beaufschlagt ist, und einem Kollektorwiderstand (10) besteht, dessen vom Kollektor abgewandter Anschluß an eine Spannungsquelle (11) angeschlossen ist.

9. Verfahren nach einem der vorhergehenden Ansprüche, dadurch gekennzeichnet,

daß die verschiedenen Belastungszustände dadurch erzielt werden, daß die Sauerstoffmeßsonde abwechselnd mit einem ersten Widerstand und mit der Parallelschaltung aus dem ersten und einem zweiten Widerstand belastet wird.

10. Verfahren nach einem der vorhergehenden Ansprüche, dadurch gekennzeichnet, daß der vorgegebene Wert einem Speicher entnommen wird, dessen Inhalt veränderbar ist.

11. Schaltungsanordnung zur Durchführung des Verfahrens nach einem der vorhergehenden Ansprüche, dadurch gekennzeichnet, daß der Sauerstoffmeßsonde (1) ein erster Widerstand (11) und eine Reihenschaltung aus einem zweiten Widerstand (10) und einem von einem Mikrocomputer (5) steuerbaren Schalter (9) parallelgeschaltet ist und daß an die Sauerstoffmeßsonde (1) ferner der Eingang (3) eines Analog/Digital-Wandlers (4) angeschlossen ist, der dem Mikrocomputer (5) zugeordnet ist.

12. Schaltungsanordnung nach Anspruch 11, dadurch gekennzeichnet, daß zwischen der Sauerstoffmeßsonde (1) und dem Eingang (3) des Analog/Digital-Wandlers (4) ein Impedanz-Wandler (2) angeordnet ist.

13. Schaltungsanordnung nach einem der Ansprüche 11 oder 12, dadurch gekennzeichnet, daß der steuerbare Schalter (9) im wesentlichen aus einem Transistor besteht.

FIG. 1

# FIG. 2